# EUROPEAN PATENT APPLICATION

(11) **EP 4 342 382 A1**
(43) Date of publication of application: **27.03.2024**
(21) Application number: 22208217.4
(22) Date of filing: 18.11.2022
(51) Int. Cl.: A61B 6/00

(54) **PROVIDING PLAQUE DATA FOR A PLAQUE DEPOSIT IN A VESSEL**

(30) Priority: 21.09.2022 US 202263408518 P
(71) Applicant: Koninklijke Philips N.V., 5656 AG Eindhoven (NL)
(72) Inventor: SCHMITT, Holger, Eindhoven (NL); VAN DER HORST, Arjen, Eindhoven (NL); GRASS, Michael, 5656AG Eindhoven (NL); HAASE, Hans Cristian, 5656AG Eindhoven (NL); NICKISCH, Hannes, Eindhoven (NL); VEMBAR, Manindranath, Eindhoven (NL)
(74) Representative: Philips Intellectual Property & Standards

(57) **Abstract**

A computer-implemented method of providing plaque data (110) for a plaque deposit (120) in a vessel (130), is provided. The method includes: receiving (S110) computed tomography, CT, data (140) representing the vessel (130); generating (S120), from the CT data (140), a cross-sectional representation (150) of the vessel (130) at each of a plurality of positions (A - A', B - B') along the vessel; extracting (S130), from the cross-sectional representations, plaque data (110) comprising at least one measurement of the plaque deposit (120) at the plurality of positions along the vessel; and outputting (S140) a graphical representation of the plaque data (110).

## Description

### TECHNICAL FIELD

The present disclosure relates to providing plaque data for a plaque deposit in a vessel. A computer-implemented method, a computer program product, and a system, are disclosed.

### BACKGROUND

Atherosclerosis is a leading cause of global mortality and morbidity. According to the WHO report "Global Health Estimates 2016: Deaths by Cause, Age, Sex, by Country and by Region, 2000-2016", Geneva, World Health Organization, 2018, cardiovascular diseases, principally atherosclerosis, are responsible for approximately 30% of world deaths. Atherosclerosis is an underlying cause of cardiovascular disease "CVD" in which plaques that are made up of fat, cholesterol, calcium, fibrin, and other substances build up in the walls of arteries. These plaques cause the arteries to harden and narrow, restricting the blood flow and supply of oxygen to vital organs, increasing the risk of blood clots that could potentially block the flow of blood to the heart or brain. Over time, the plaque may open, or rupture, forming a thrombus, or blood clot, further restricting the blood flow. The thrombus may also break away as an embolus. The embolus can become lodged elsewhere in the body and form an embolism that likewise blocks the artery. Thromboses may occur in various parts of the body, including in the heart and the brain, where their effects can be severe unless treated quickly. In the brain, for example, a thrombus, or an embolism, can lead to conditions such as (ischemic) stroke.

Various intravascular treatment devices are available for treating vascular plaque. These include an intravascular lithotripsy "IVL" balloon that delivers shock wave pulses to deliver to the vessel in order to break-up the plaque, a laser atherectomy catheter that uses laser irradiation to break-up the plaque, an orbital atherectomy device that performs an orbital sanding operation around the vessel axis in order to break-up the plaque, a rotablation atherectomy device that performs a rotational sanding or cutting operation around the vessel axis in order to break-up the plaque, and a scoring or cutting balloon that includes a blade or a wire on the outer surface of a balloon and which is rotated within the vessel in order to scrape plaque from the vessel wall. An example of a commercial IVL balloon is the Shockwave C2 Coronary IVL Catheter marketed by Shockwave Medical, Santa Clara, USA. An example of a commercial laser atherectomy catheter is the Turbo-Elite laser atherectomy catheter marketed by Philips Healthcare, Best, The Netherlands. An example of a commercial orbital atherectomy device is the Diamondback 360 Coronary orbital atherectomy system marketed by Cardiovascular Systems Inc., Minneapolis, USA. An example of a commercial rotablation atherectomy device is the Peripheral Rotablator rotational atherectomy system marketed by Boston Scientific, Massachusetts, USA. An example of a commercial scoring or cutting balloon is the Advance Enforcer 35 Focal-Force PTA Balloon Catheter marketed by Cook Medical, Limerick, Ireland.

Intravascular ultrasound "IVUS" imaging is currently considered to be the gold standard for assessing vascular plaque, as well as for planning its treatment. Optical coherence tomography "OCT" imaging is also often used. Both IVUS, and OCT, provide comprehensive information on the distribution of plaque around and along a vessel. However, the adoption of these imaging techniques faces challenges due to their size, cost, and the steep learning curve to image interpretation.

Consequently, there remains a need to provide improved techniques for assessing vascular plaque. There also remains a need to provide improved techniques for planning, and also guiding vascular plaque treatment procedures. This is because after having selected a treatment device to treat a plaque deposit, it can be challenging to determine the values of parameters to use with the device. After having selected a treatment device, values typically need to be determined for parameters such as the size of treatment device, the location of the treatment device in the vessel, and also the amount of treatment to deliver with the treatment device.

### SUMMARY

According to one aspect of the present disclosure, a computer-implemented method of providing plaque data for a plaque deposit in a vessel, is provided. The method includes:
receiving computed tomography, CT, data representing the vessel;
generating, from the CT data, a cross-sectional representation of the vessel at each of a plurality of positions along the vessel;
extracting, from the cross-sectional representations, plaque data comprising at least one measurement of the plaque deposit at the plurality of positions along the vessel; and
outputting a graphical representation of the plaque data.

Since, in the above method, the plaque data is provided from CT data, the method obviates challenges to the adoption of the current gold standard technique for assessing vascular plaque, IVUS. CT data is also often already available for the vasculature for patients who are subject to vascular plaque, and consequently the use of CT data in the method obviates the need to acquire additional imaging data for the subject. Moreover, since the plaque data is extracted from cross sectional representations of the vessel in the CT data, the method provides accurate measurements of the plaque deposit along the vessel.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a vessel 130 with a plaque deposit 120, in accordance with some aspects of the present disclosure.
Fig. 2 is a flowchart illustrating an example of a computer-implemented method of providing plaque data for a plaque deposit in a vessel, in accordance with some aspects of the present disclosure.
Fig. 3 is a schematic diagram illustrating an example of a system 200 for providing plaque data for a plaque deposit in a vessel, in accordance with some aspects of the present disclosure.
Fig. 4 illustrates a volumetric image that is reconstructed from CT data 140 representing a vessel 130, in accordance with some aspects of the present disclosure.
Fig. 5 illustrates a cross-sectional representation 150 of a vessel 130 at a position A - A' along the vessel, in accordance with some aspects of the present disclosure.
Fig. 6 is a schematic diagram illustrating a cross-sectional representation 150 of a vessel 130 including a first example of a measurement of a plaque deposit 120 in the form of an angular extent f of the plaque deposit around a centerline 160 of the vessel 130, in accordance with some aspects of the present disclosure.
Fig. 7 is a schematic diagram illustrating a cross-sectional representation 150 of a vessel 130 including a second example of a measurement of a plaque deposit 120 in the form of an angular extent f of the plaque deposit around a centerline 160 of the vessel 130, in accordance with some aspects of the present disclosure.
Fig. 8 is a schematic diagram illustrating an example of an intravascular treatment device 180 in the form of an IVL balloon for use in an intravascular plaque treatment procedure, in accordance with some aspects of the present disclosure.
Fig. 9 is a flowchart illustrating an example of a computer-implemented method of planning an intravascular plaque treatment procedure, in accordance with some aspects of the present disclosure.
Fig. 10 is a flowchart illustrating a first example of a computer-implemented method of providing guidance during an intravascular plaque treatment procedure, in accordance with some aspects of the present disclosure.
Fig. 11 is a schematic diagram illustrating an example of a system 300 for providing guidance during an intravascular plaque treatment procedure, in accordance with some aspects of the present disclosure.
Fig. 12 is a flowchart illustrating a second example of a computer-implemented method of providing guidance during an intravascular plaque treatment procedure, in accordance with some aspects of the present disclosure.

### DETAILED DESCRIPTION

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to examples of a computer-implemented method of providing plaque data for a plaque deposit in a vessel. In general, the vessel may be in any anatomical region, and the vessel may be any type of vessel. Thus, the vessel may be an artery, or a vein, and the artery, or vein, may be in any location in the body, such as in the heart, the brain, the arm, the leg, and so forth.

In some examples, reference is made to the provision of plaque data for a plaque deposit in the form of a mature plaque. However, it is to be appreciated that this type of plaque serves only as an example, and that the methods disclosed herein may be used to provide plaque data for a plaque deposit that is in a different stage of maturity. For instance, the plaque may be a so-called fatty streak, or a ruptured plaque. The methods disclosed may also be used to provide plaque data for plaque deposits that have a different composition to a mature plaque.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

As mentioned above, there remains a need to provide improved techniques for assessing vascular plaque.

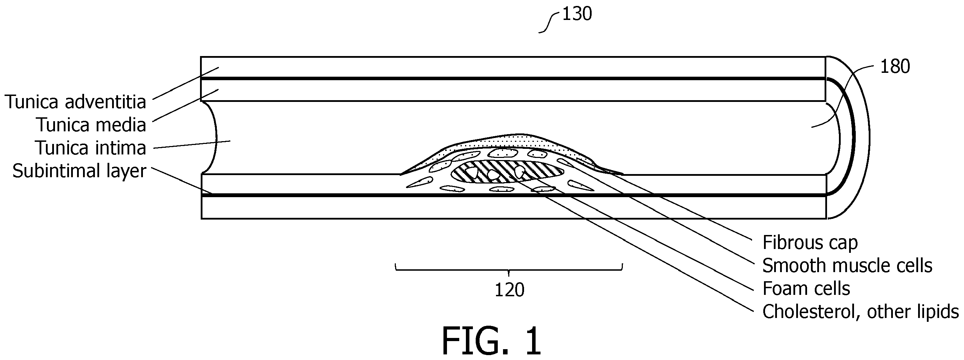

Fig. 1 is a schematic diagram illustrating an example of a vessel 130 with a plaque deposit 120, in accordance with some aspects of the present disclosure. The plaque deposit 120 illustrated in Fig. 1 shows a so-called mature plaque, and includes a fibrous cap. The fibrous cap is formed of intimal smooth muscle cells and connective tissue. The fibrous cap separates the lumen of the vessel from the thrombogenic core of the plaque, and as such is the final barrier to thrombus formation. The core of the plaque illustrated in Fig. 1 includes foam cells, cholesterol, and other lipids.

Over time, the composition, and also distribution, of a plaque in a vessel can change. For instance, the plaque may start with a so-called fatty streak, and subsequently evolve into the mature plaque illustrated in Fig. 1. The mature plaque may subsequently evolve further by rupturing, resulting in a thrombus. During its evolution, vascular calcifications can occur in the vessel 130. As mentioned in a document by Lee, S., et al., Vascular Calcification-New Insights Into Its Mechanism, Int. J. Mol. Sci. 2020, 21, 2685, pages 1 - 33, vascular calcification is defined as mineral deposition, in the vasculature, in a form of calcium-phosphate complexes. Vascular calcification can be classified into two forms, depending on where the mineral is deposited. Vascular calcification occurs in the intimal and medial layers. Intimal calcification is associated with atherosclerotic plaques and is known as the result of lipid accumulation, macrophage invasion, proliferation of smooth muscle cells, and dysfunction of extracellular matrix proteins, in response to chronic arterial inflammation. These unstable and rupturable plaques form on the inner blood vessel wall, causing obstructive vascular disease. Medial calcification is associated with aging, diabetes mellitus, hypertension, osteoporosis, and chronic kidney disease. Medial calcification can occur without vascular stenosis, which mainly causes vascular stiffness, and increases the incidence of cardiovascular complications. The medial layer of the vessel wall is composed of smooth muscle cells and the elastin-rich extracellular matrix. In medial calcification, the process of differentiation of smooth muscle cells into osteoblast-like cells is akin to bone formation. Thus, in order to assess the risk of medical complications such as those mentioned above, there is a need to assess vascular plaques.

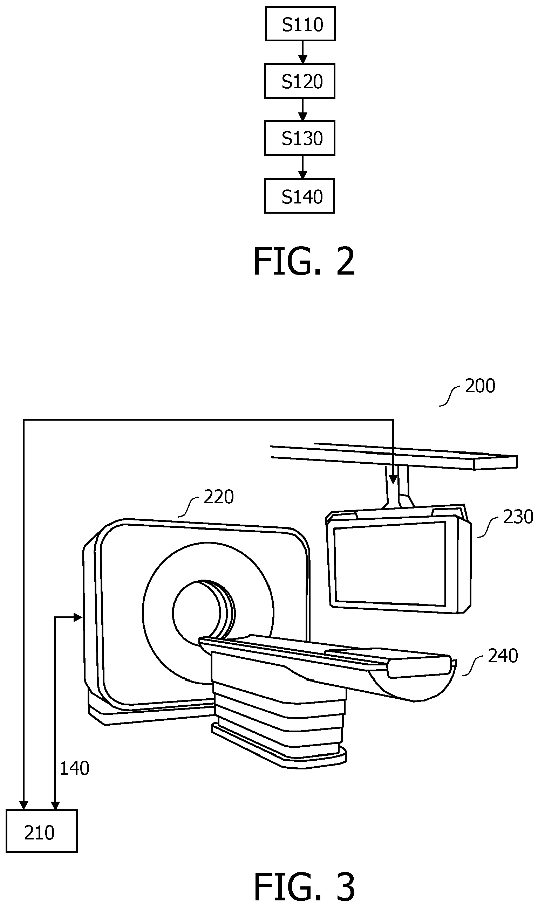

Fig. 2 is a flowchart illustrating an example of a computer-implemented method of providing plaque data for a plaque deposit in a vessel, in accordance with some aspects of the present disclosure. Fig. 3 is a schematic diagram illustrating an example of a system 200 for providing plaque data for a plaque deposit in a vessel, in accordance with some aspects of the present disclosure. The system 200 includes one or more processors 210. It is noted that operations described in relation to the method illustrated in Fig. 2, may also be performed by the one or more processors 210 of the system 200 illustrated in Fig. 3. Likewise, operations described in relation to the one or more processors 210 of the system 200, may also be performed in the method described with reference to Fig. 2. With reference to Fig. 2, the computer-implemented method of providing plaque data 110 for a plaque deposit 120 in a vessel 130, includes:
receiving S110 computed tomography, CT, data 140 representing the vessel 130;
generating S120, from the CT data 140, a cross-sectional representation 150 of the vessel 130 at each of a plurality of positions A - A', B - B' along the vessel;
extracting S130, from the cross-sectional representations, plaque data 110 comprising at least one measurement of the plaque deposit 120 at the plurality of positions along the vessel; and
outputting S140 a graphical representation of the plaque data 110.

Since, in the above method, the plaque data is provided from CT data, the method obviates challenges to the adoption of the current gold standard technique for assessing vascular plaque, IVUS. CT data is also often already available for the vasculature for patients who are subject to vascular plaque, and consequently the use of CT data in the method obviates the need to acquire additional imaging data for the subject. Moreover, since the plaque data is extracted from cross sectional representations of the vessel in the CT data, the method provides accurate measurements of the plaque deposit along the vessel.

With reference to the method illustrated in Fig. 2, in the operation S110, CT data 140 representing the vessel 130, is received.

The CT data 140 that is received in the operation S110 may in general represent a static image of the vessel 130, or it may represent a temporal sequence of images of the vessel 130. In the latter case, the temporal sequence of images may be generated substantially in real-time, and the method described above may be performed substantially in real-time. Consequently, the graphical representation of the plaque data may be outputted in real-time.

In general, the CT data 140 that is received in the operation S110 may be raw data, i.e. data that has not yet been reconstructed into a volumetric, or 3D, image, or it may be image data, i.e. data that has already been reconstructed into a volumetric image. The CT data may also be referred-to as volumetric data. The CT data 140 may be generated by a CT imaging system, or, as described in more below, it may be generated by rotating, or stepping the X-ray source and X-ray detector of an X-ray projection imaging system around the vessel.

A CT imaging system generates CT data by rotating, or stepping, an X-ray source-detector arrangement around an object and acquiring X-ray attenuation data for the object from multiple rotational angles with respect to the object. The CT data may then be reconstructed into a 3D image of the object. Examples of CT imaging systems that may be used to generate the CT data 140 include cone beam CT imaging systems, photon counting CT imaging systems, dark-field CT imaging systems, and phase contrast CT imaging systems. An example of a CT imaging system 220 that may be used to generate the CT data 140 that is received in the operation S110, is illustrated in Fig. 3. By way of an example, the CT data 140 may be generated by the CT 5000 Ingenuity CT scanner that is marketed by Philips Healthcare, Best, The Netherlands.

As mentioned above, the CT data 140 that is received in the operation S 110 may alternatively be generated by rotating, or stepping the X-ray source and X-ray detector of an X-ray projection imaging system around the vessel. An X-ray projection imaging system typically includes a support arm such as a so-called "C-arm" that supports an X-ray source and an X-ray detector. X-ray projection imaging systems may alternatively include a support arm with a different shape to this example, such as an O-arm, for example. In contrast to a CT imaging system, an X-ray projection imaging system generates X-ray attenuation data for an object with the X-ray source and X-ray detector in a static position with respect to the object. The X-ray attenuation data may be referred-to as projection data, in contrast to the volumetric data that is generated by a CT imaging system. The X-ray attenuation data that is generated by an X-ray projection imaging system is typically used to generate a 2D image of the object. However, an X-ray projection imaging system may generate CT data, i.e. volumetric data, by rotating, or stepping, its X-ray source and X-ray detector around an object and acquiring projection data for the object from multiple rotational angles with respect to the object. Image reconstruction techniques may then be used to reconstruct the projection data that is obtained from the multiple rotational angles into a volumetric image in a similar manner to the reconstruction of a volumetric image using X-ray attenuation data that is acquired from a CT imaging system. Thus, the CT data 140 that is received in the operation S110, may be generated by a CT imaging system, or alternatively, it may be generated by an X-ray projection imaging system. An example of an X-ray projection imaging system that may be used to generate the CT data 140, is the Azurion 7 X-ray projection imaging system that is marketed by Philips Healthcare, Best, The Netherlands.

In some examples, the CT data 140 that is received in the operation S110 is spectral CT data. The spectral CT data defines X-ray attenuation of the vessel in a plurality of different energy intervals DE_{1..m}. In general there may be two or more energy intervals; i.e. *m* is an integer, and *m* ≥ 2. In this regard, the spectral CT data 140 that is received in the operation S110 may be generated by a spectral CT imaging system, or by a spectral X-ray projection imaging system. In the latter case, the spectral CT data may be acquired by rotating, or stepping the X-ray source and X-ray detector of the spectral X-ray projection imaging system around the vessel, as described above. More generally, the spectral CT data 140 that is received in the operation S110 may be generated by a spectral X-ray imaging system.

The ability to generate X-ray attenuation data at multiple different energy intervals DE_{1..m} distinguishes a spectral X-ray imaging system from a conventional X-ray imaging system. By processing the data from the multiple different energy intervals, a distinction can be made between media that have similar X-ray attenuation values when measured within a single energy interval, and which would be indistinguishable in conventional X-ray attenuation data. Examples of spectral X-ray imaging systems that may be used to generate spectral CT data 140 that is received in the operation S110 include cone beam spectral X-ray imaging systems, photon counting spectral X-ray imaging systems, dark-field spectral X-ray imaging systems, and phase contrast spectral X-ray imaging systems. An example of a spectral CT imaging system that may be used to generate spectral CT data 140 that is received in the operation S110 is the Spectral CT 7500 that is marketed by Philips Healthcare, Best, The Netherlands.

In general, spectral CT data 140 may be generated by various different configurations of spectral X-ray imaging systems that include an X-ray source and an X-ray detector. The X-ray source of a spectral X-ray imaging system may include multiple monochromatic sources, or one or more polychromatic sources, and the X-ray detector of a spectral X-ray imaging system may include: a common detector for detecting multiple different X-ray energy intervals, or multiple detectors wherein each detector detects a different X-ray energy interval DE_{1..m}, or a multi-layer detector in which X-rays having energies within different X-ray energy intervals are detected by corresponding layers, or a photon counting detector that bins detected X-ray photons into one of multiple energy intervals based on their individual energies. In a photon counting detector, the relevant energy interval may be determined for each received X-ray photon by detecting the pulse height induced by electron-hole pairs that are generated in response to the X-ray photon's absorption in a direct-conversion material.

Various configurations of the aforementioned X-ray sources and detectors may be used to detect X-rays within different X-ray energy intervals DE_{1..m}. In general, discrimination between different X-ray energy intervals may be provided at the source by temporally switching the X-ray tube potential of a single X-ray source, i.e. "rapid kVp switching", or by temporally switching, or filtering, the emission of X-rays from multiple X-ray sources. In such configurations, a common X-ray detector may be used to detect X-rays across multiple different energy intervals, X-ray attenuation data for each energy interval being generated in a time-sequential manner. Alternatively, discrimination between different X-ray energy intervals may be provided at the detector by using a multi-layer detector, or a photon counting detector. Such detectors can detect X-rays from multiple X-ray energy intervals DE_{1..m} near-simultaneously, and thus there is no need to perform temporal switching at the source. Thus, a multi-layer detector, or a photon counting detector, may be used in combination with a polychromatic source to generate X-ray attenuation data at different X-ray energy intervals DE_{1..m}.

Other combinations of the aforementioned X-ray sources and detectors may also be used to provide the spectral CT data 140. For example, in a yet further configuration, the need to sequentially switch different X-ray sources emitting X-rays at different energy intervals may be obviated by mounting X-ray source-detector pairs to a gantry at rotationally-offset positions around an axis of rotation. In this configuration, each source-detector pair operates independently, and separation between the spectral CT data for different energy intervals DE_{1..m} is facilitated by virtue of the rotational offsets of the source-detector pairs. Improved separation between the spectral CT data for different energy intervals DE_{1..m} may be achieved with this configuration by applying an energy-selective filter to the X-ray detector(s) in order to reduce the effects of X-ray scatter.

In general, the CT data 140 that is received in the operation S 110 may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals. The CT data 140 that is received in the operation S110 may be received from various sources. For example, the CT data 140 may be received from an imaging system, such as one of the imaging systems described above. Alternatively, the CT data 140 may be received from another source, such as a computer readable storage medium, the Internet, or the Cloud, for example.

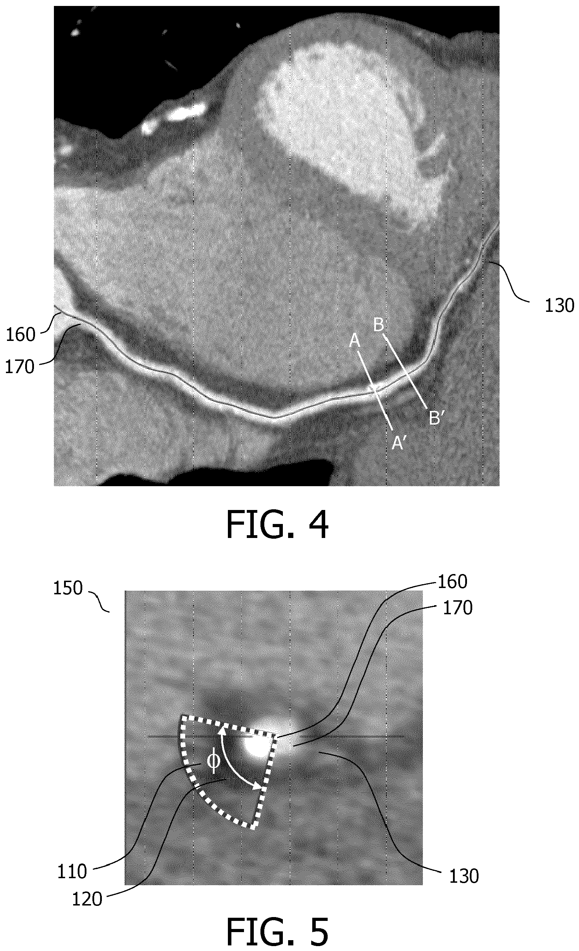

An example of the CT data 140 that is received in the operation S110 is described with reference to Fig. 4, which illustrates a volumetric image that is reconstructed from CT data 140 representing a vessel 130, in accordance with some aspects of the present disclosure. The volumetric image illustrated in Fig. 4 represents a vessel 130 in the form of a coronary artery. It is noted that the volumetric image appears to be planar due to the limitations on its reproduction.

Returning to the method illustrated in Fig. 2, in the operation S120, a cross-sectional representation 150 of the vessel 130 is generated from the CT data 140 at each of a plurality of positions A - A', B - B' along the vessel. Each cross-sectional representation 150 represents the X-ray attenuation in a transverse slice through an axis of the vessel. The transverse slices may be arranged perpendicularly with respect to the axis of the vessel. The cross sectional representations may be generated from the CT data 140 using known image processing techniques. By way of an example, Fig. 5 illustrates a cross-sectional representation 150 of a vessel 130 at a position A - A' along the vessel, in accordance with some aspects of the present disclosure. The cross-sectional representation 150 illustrated in Fig. 5 is generated at the position A - A' in Fig. 4. In the operation S120, a cross-sectional representation of the vessel 130 is generated at one or more other positions along the vessel. For instance, a further cross-sectional representation may be generated at the position B - B' in Fig. 4. The positions may be distributed at various intervals along the vessel. The cross-sectional representations may be distributed at regular intervals along the vessel 130. For example, the cross-sectional representations may be distributed at intervals that are below 1 millimeter, or intervals that are 5 millimeters or more. For instance, the cross-sectional representations may be generated at intervals of 1 millimeter, or at intervals of 5 millimeters, and so forth. Alternatively, the positions may be distributed at varying intervals. In some examples, the size of the intervals may depend upon the dimensions of the vessel.

In one example, the operation of generating S120 a cross-sectional representation 150 of the vessel 130 at each of a plurality of positions A - A', B - B' along the vessel, includes identifying, from the CT data 140, a centerline 160 of the vessel 130, and the cross-sectional representations of the vessel are generated at the plurality of positions along the centerline 160 of the vessel. By way of an example, the centerline 160 of the vessel 130 is illustrated in Fig. 4. In this example, each cross-sectional representation 150 represents the X-ray attenuation in a transverse slice through the centerline of the vessel. The transverse slices may be arranged perpendicularly with respect to the centerline of the vessel.

In one example, the operation of identifying a centerline 160 of the vessel 130, comprises determining a centerline of the lumen 170 of the vessel, and defining the centerline 160 of the vessel 130 as the centerline of the lumen 170. The lumen may be considered to provide a more accurate position of the centerline of the vessel. This operation may be performed by reconstructing a volumetric image from the CT data 140, and segmenting the reconstructed volumetric image in order to identify the lumen 170 of the vessel 140. Various segmentation algorithms may be used for this purpose, including model-based segmentation, watershed-based segmentation, region growing, level sets, graphcuts, and so forth. A neural network may also be trained to segment the reconstructed volumetric image in order to identify the lumen 170 of the vessel 140.

In one example, the CT data 140 that is received in the operation S110 comprises spectral CT data defining X-ray attenuation of the vessel 130 in a plurality of different energy intervals DE_{1..m}. In this example, the operation of determining a centerline of the lumen 170 of the vessel 130 comprises applying a material decomposition algorithm to the spectral CT data. In this example, the material decomposition algorithm is applied to the spectral CT data in order to identify the lumen, and the centerline of the lumen 170 may then be identified, and used as the centerline of the vessel. As mentioned above, the use of spectral CT data facilitates a distinction between media that have similar X-ray attenuation values when measured within a single energy interval, and which would be indistinguishable in conventional X-ray attenuation data. In this example, the application of a material decomposition algorithm to the spectral CT data provides improved distinction between the lumen, which may be filled with blood or a contrast agent that includes a material such as Iodine, and the surrounding tissue, which is formed from different materials. Consequently, this example facilitates a more accurate definition of the centerline 160 of the lumen 170.

Various material decomposition algorithms may be applied to the spectral CT data, in accordance with this example. One example of a material decomposition algorithm that may be used is disclosed in a document by Brendel, B. et al., "Empirical, projection-based basis-component decomposition method", Medical Imaging 2009, Physics of Medical Imaging, edited by Ehsan Samei and Jiang Hsieh, Proc. of SPIE Vol. 7258, 72583Y. Another example of a material decomposition algorithm that may be used is disclosed in a document by Roessl, E. and Proksa, R., "K-edge imaging in X-ray computed tomography using multi-bin photon counting detectors", Phys Med Biol. 2007 Aug 7, 52(15):4679-96. Another example of a material decomposition algorithm that may be used is disclosed in the published PCT patent application WO/2007/034359 A2.

Returning to the method illustrated in Fig. 2, in the operation S130, plaque data 110 is extracted from the cross-sectional representations. The plaque data comprises at least one measurement of the plaque deposit 120 at the plurality of positions along the vessel.

In general, the extraction of the plaque data from the cross-sectional representations may be performed based on differences in X-ray attenuation in the cross-sectional representations. If the CT data 140 is conventional CT data, the plaque data may be extracted using image processing techniques such as segmentation. Plaque deposits also tend to have a different level of X-ray attenuation to that of the vessel lumen. Plaque deposits from e.g. calcium tend to have a higher Hounsfield Units attenuation than vessel wall, and consequently, segmentation may be used to delineate features in the cross-sectional representations. If, however, the CT data 140 comprises spectral CT data defining X-ray attenuation of the vessel 130 in a plurality of different energy intervals DE_{1..m}, the operation of extracting S130 plaque data 110 may be performed by applying a material decomposition algorithm to the spectral CT data. As mentioned above, as compared to the use of conventional CT data, the use of spectral CT data and a material decomposition algorithm facilitates improved distinction between the plaque data and surrounding tissue. The various material decomposition algorithms described above may be used for this purpose.

In one example, the operation of extracting S130 plaque data 110 comprises applying a material decomposition algorithm to the spectral CT data to identify a type of the plaque deposit 120 at the plurality of positions A - A', B - B' along the vessel 130. Different types of plaque may be identified based on the presence of materials that are present in the plaque. Examples of types of plaque that may be identified in accordance with this example include soft plaque, mixed plaque, and calcified plaque. The identification of the types of plaque deposit may be used by a physician to determine a level of risk associated with the plaque such as its vulnerability to rupture, or to determine how to treat the plaque, for example.

Various measurements of the plaque deposit 120 may be extracted from the cross-sectional representations in the operation S130. For instance, the at least one measurement of the plaque deposit 120 may include at least one of: a thickness of the plaque deposit, a depth of the plaque deposit from a centerline 160 of the vessel 130, an angular extent f of the plaque deposit 120 around a centerline 160 of the vessel 130, and an eccentricity of the plaque deposit. By way of an example, the thickness of the plaque deposit may be calculated as the longest radial path extending from the centerline of the lumen through the plaque. The thickness of the plaque deposit may also be calculated in other ways, such as the shortest radial path extending from the centerline of the lumen through the plaque. The depth of the plaque deposit from a centerline 160 of the vessel 130 may define the depth of a plaque deposit in the intimal layer, or in the medial layer. The angular extent f of the plaque deposit 120 around a centerline 160 of the vessel 130 may be defined as the angular extent of its largest connected component in the cross section, for example.

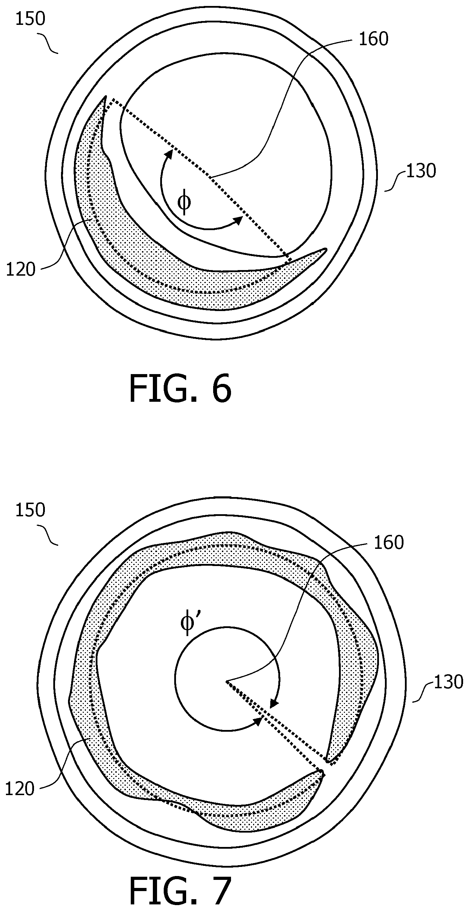

By way of an example, Fig. 6 is a schematic diagram illustrating a cross-sectional representation 150 of a vessel 130 including a first example of a measurement of a plaque deposit 120 in the form of an angular extent f of the plaque deposit around a centerline 160 of the vessel 130, in accordance with some aspects of the present disclosure. In Fig. 6, the angular extent of the plaque deposit is less than 180 degrees.

By way of another example, Fig. 7 is a schematic diagram illustrating a cross-sectional representation 150 of a vessel 130 including a second example of a measurement of a plaque deposit 120 in the form of an angular extent f of the plaque deposit around a centerline 160 of the vessel 130, in accordance with some aspects of the present disclosure. In Fig. 7, the angular extent f of the plaque deposit is approximately 360 degrees. Another example of the angular extent f of the plaque deposit around a centerline 160 of the vessel 130 is illustrated in Fig. 5. The eccentricity of the plaque deposit may be determined by fitting an ellipse to the plaque deposit and calculating the eccentricity of the ellipse.

In one example, the plaque data 110 that is extracted in the operation S130 includes an estimation of a length of the plaque deposit 120 along the vessel 130. The estimation of the length of the plaque deposit 120 along the vessel 130 is calculated based on a separation between the positions along the vessel corresponding to the proximal and distal ends of a contiguous set of cross-sectional representations in which at least one measurement of the plaque deposit 120 exceeds a predetermined value.

In this example, the measurement of the plaque deposit may be a thickness of the plaque deposit, or an angular extent f of the plaque deposit 120 around a centerline 160 of the vessel 130, for example. The predetermined value of the measurement sets a threshold that is used to identify cross sectional representations that are likely to represent the same plaque deposit. Consequently, the proximal and distal ends of the contiguous set of cross-sectional representations, define the length of the plaque deposit. By way of an example, the predetermined value of the angular extent f of the plaque deposit 120 around a centerline 160 of the vessel 130, may be defined as 180 degrees, and consequently the length of the plaque deposit 120 would be defined as the length of the vessel between the proximal and distal ends of a contiguous set of cross sectional representations in which the angular extent f of the plaque deposit 120 exceeds 180 degrees.

Returning to the method illustrated in Fig. 2, in the operation S140, a graphical representation of the plaque data 110 is outputted. The plaque data 110 may be outputted in various ways. In some examples, the plaque data 110 is outputted to a display. The plaque data 110 may be outputted to the display 230 illustrated in Fig. 3, for example. The graphical representation of the plaque data 110 may be outputted to the display 230 in any human-readable format. For example, the plaque data may be outputted in the form or an image, or an icon, or in text, or in a numerical format. In some examples, the plaque data 110 is outputted in combination with a graphical representation of the CT data 140 and/or the cross-sectional representations. For instance, the plaque data 110 may be overlaid onto the graphical representation of the CT data 140 and/or the cross-sectional representations. For example, if the measurement of the plaque deposit 120 includes an angular extent f of the plaque deposit 120 around a centerline 160 of the vessel 130, a graphical representation of the plaque data 110 may be outputted as an overlay on the corresponding cross-sectional representation 150, as illustrated in Fig. 5. Other measurements of the plaque deposit 120 may be outputted in a similar manner as an overlay on the corresponding cross-sectional representation 150. The measurements of the plaque deposit 120 may alternatively be outputted graphically in a different manner, such as in a table, for example.

In one example, both the cross-sectional representations as well as the graphical representation of the CT data 140, are outputted to the display 230. In this example, the graphical representation of the CT data 140 is provided in the form of a reconstructed volumetric image, or a planar image. If the CT data 110 comprises spectral CT data and a material decomposition algorithm is applied to the spectral CT data to identify a type of the plaque deposit 120, the type of the plaque deposit may be identified in the cross sectional representations. For instance, a color coding, or a shading, may be applied to the cross sectional representations in order to identify the type of the plaque deposit. A marker may be provided in the graphical representation of the CT data 140 to identify the position, e.g. A-A', at which the displayed cross sectional representation is generated. A user-operable control such as a slider control may be provided that allows the user to select a position in the graphical representation of the CT data 140 to generate the displayed cross sectional representation.

In addition to the plaque data described above, other types of data may also be extracted from the CT data 110.

In one example, the method described with reference to Fig. 2 also includes extracting, from the cross-sectional representations, lumen data and/or vessel wall data for the vessel 130, the lumen data comprising at least one measurement of a lumen 170 of the vessel 130, and the vessel wall data comprising at least one measurement of the wall of the vessel 130, at the plurality of positions A - A', B - B' along the vessel.

The lumen data that is extracted in this example may for instance include a measurement of an area of the lumen, or a minimum or maximum diameter of the lumen, or a measurement of a shape of the lumen. The shape of the lumen may for instance be an eccentricity of the lumen. The eccentricity may be calculated in the same manner as described above for the plaque data. The vessel wall data that is extracted in this example may for instance include a measurement of a thickness of the vessel wall, or an area encompassed by the vessel wall, or a minimum or maximum diameter encompassed by the vessel wall. The lumen data and/or vessel wall data can be outputted to the display 230 and used by a physician to perform a diagnosis on the vessel 130.

In this example, the lumen data may be extracted in a similar manner to the plaque data described above. In other words, if the CT data 140 is conventional CT data, the plaque data may be extracted using image processing techniques such as segmentation. If, however, the CT data 140 comprises spectral CT data defining X-ray attenuation of the vessel 130 in a plurality of different energy intervals DE_{1..m}, the operation of extracting lumen data for the vessel 130, may be performed by applying a material decomposition algorithm to the spectral CT data. As mentioned above, as compared to the use of conventional CT data, the use of spectral CT data and a material decomposition algorithm facilitates improved distinction between the lumen and surrounding tissue. The various material decomposition algorithms described above may be used for this purpose.

In many situations, the lumen in a cross sectional representation has a reduced size due to the presence of a plaque deposit. In such situations, a physician is often interested in knowing the size of the lumen in the absence of the plaque deposit. This helps the physician to determine the impact of the plaque deposit on blood flow, and consequently to assess the benefit of treating the plaque deposit. In one example, an estimated plaque-free measurement of the lumen 170 for a cross sectional representation is determined from a neighboring cross-sectional representation. In this example, the method described with reference to Fig. 2 includes:
estimating, for cross-sectional representations wherein at least one measurement of the plaque deposit 120 exceeds a threshold value, a plaque-free measurement of the lumen 170 at the corresponding position along the vessel 130; and
wherein the estimated plaque-free measurement of the lumen 170 is determined based on the corresponding measurement of the lumen from a neighboring cross-sectional representation wherein the at least one measurement of the plaque deposit 120 is below the threshold value.

By way of an example, a physician may be interested in knowing what the lumen area would be for a cross sectional representation in the absence of a plaque deposit. In this case, the threshold value of the angular extent f of the plaque deposit 120 around a centerline 160 of the vessel 130 might be set to 90 degrees, and the estimated plaque-free lumen area for a cross-sectional representation might be obtained from a neighboring cross-sectional representation in which the angular extent f of the plaque deposit 120 around a centerline 160 of the vessel 130 is less than 90 degrees. The neighboring cross section may be the closest neighboring cross sectional representation for which the condition applies. Alternatively, the neighboring cross sectional representation may be a cross sectional representation that is predetermined number of cross-sectional representations, or a predetermined distance, away from the closest neighboring cross sectional representation for which the condition applies.

In another example, a computer program product is provided. The computer program product comprises instructions which when executed by one or more processors, cause the one or more processors to carry out a method of providing plaque data 110 for a plaque deposit 120 in a vessel 130. The method comprises:
receiving S110 computed tomography, CT, data 140 representing the vessel 130;
generating S120, from the CT data 140, a cross-sectional representation 150 of the vessel 130 at each of a plurality of positions A - A', B - B' along the vessel;
extracting S130, from the cross-sectional representations, plaque data 110 comprising at least one measurement of the plaque deposit 120 at the plurality of positions along the vessel; and
outputting S140 a graphical representation of the plaque data 110.

In another example, a system for providing plaque data 110 for a plaque deposit 120 in a vessel 130, is provided. The system comprises one or more processors 210 configured to:
receive S110 computed tomography, CT, data 140 representing the vessel 130;
generate S120, from the CT data 140, a cross-sectional representation 150 of the vessel 130 at each of a plurality of positions A - A', B - B' along the vessel;
extract S130, from the cross-sectional representations, plaque data 110 comprising at least one measurement of the plaque deposit 120 at the plurality of positions along the vessel; and
output S140 a graphical representation of the plaque data 110.

An example of the system 200 is illustrated in Fig. 3. It is noted that the system 200 may also include one or more of: a CT imaging system 220 for providing the CT data 140, a display 230 for displaying a graphical representation of the plaque data 110, a graphical representation of the CT data, the cross-sectional representations 150 of the vessel 130, and so forth; a patient bed 240; and a user input device configured to receive user input (not illustrated in Fig. 3), such as a keyboard, a mouse, a touchscreen, and so forth.

As mentioned above, various intravascular treatment devices are available for treating vascular plaque. These include an intravascular lithotripsy "IVL" balloon that delivers shock wave pulses to deliver to the vessel in order to break-up the plaque, a laser atherectomy catheter that uses laser irradiation to break-up the plaque, an orbital atherectomy device that performs an orbital sanding operation around the vessel axis, a rotablation atherectomy device that performs a rotational sanding or cutting operation around the vessel axis, and a scoring or cutting balloon that includes a blade or a wire on the outer surface of a balloon and which is rotated within the vessel in order to scrape plaque from vessel walls.

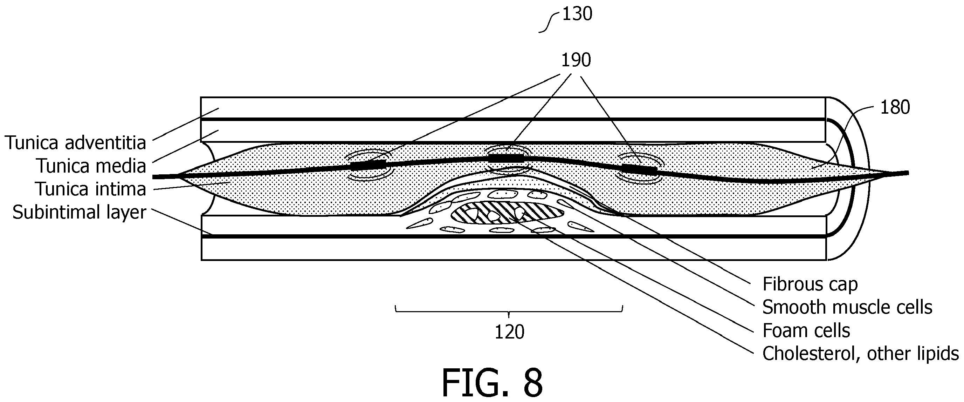

By way of an example, Fig. 8 is a schematic diagram illustrating an example of an intravascular treatment device 180 in the form of an IVL balloon for use in an intravascular plaque treatment procedure, in accordance with some aspects of the present disclosure. The IVL balloon includes a plurality of shockwave emitters 190 that are disposed on a catheter. In-use, the balloon is inflated with saline, and shock waves generated by the emitters 190 travel through the balloon to the plaque deposit 120, resulting in the fracture of calcium in the plaque deposit. The IVL balloon may be used to fracture calcium deposits in that are in the intimal and also medial layers.

As mentioned above, after having selected a treatment device to treat a plaque deposit, it can be challenging to determine the values of parameters to use with the device. For instance, values typically need to be determined for parameters such as the size of treatment device, the location of the treatment device in the vessel, and also the amount of treatment to deliver with the treatment device. With reference to the IVL balloon illustrated in Fig. 8, values may need to be determined for parameters such as a size of the IVL balloon, a location of the IVL balloon with respect to the plaque deposit 120, a number of shock wave pulses to deliver to the vessel 130 from one or more shock wave emitters 190 of the IVL balloon, an IVL balloon pressure to use during a delivery of shock waves to the vessel 130 from the IVL balloon.

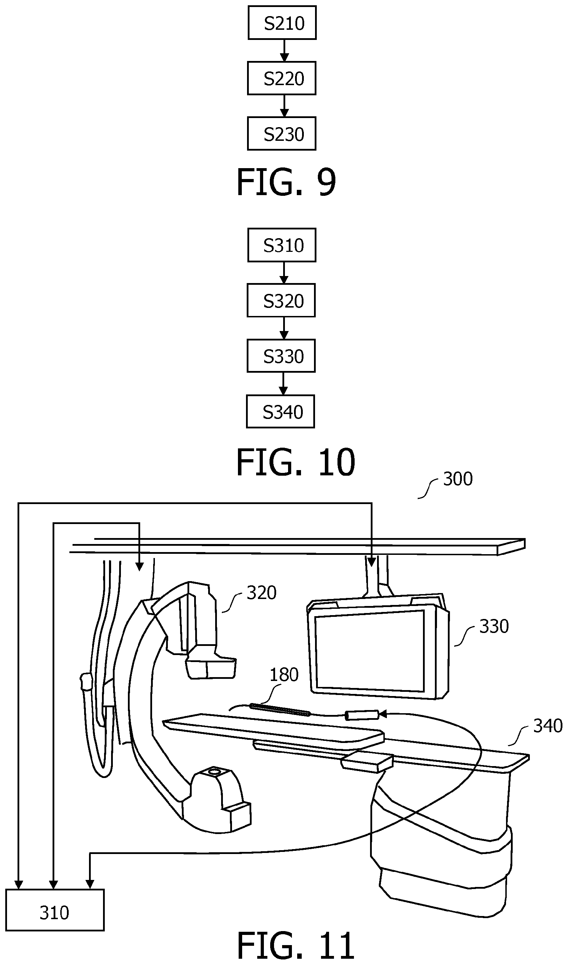

Fig. 9 is a flowchart illustrating an example of a computer-implemented method of planning an intravascular plaque treatment procedure, in accordance with some aspects of the present disclosure. With reference to Fig. 9, the method includes:
executing S210 the operations S110, S120, S130, and S140;
determining S220 a recommended value of at least one parameter of an intravascular treatment device 180 for use in the intravascular plaque treatment procedure, based on the plaque data 110; and
outputting S230 the recommended value of the at least one parameter.

Since, in this example, the recommended value of the parameter(s) are determined based on the plaque data 110, the parameter(s) are tailored to the plaque deposit. This results in a more effective treatment of the plaque deposit.

The values of various parameters may be determined in the operation S220, depending on the type of intravascular treatment device that is used. By way of an example, if the intravascular treatment device 180 comprises an intravascular lithotripsy, IVL, balloon, the at least one parameter may include one or more of: a size of the IVL balloon, a location of the IVL balloon with respect to the plaque deposit 120, a number of shock wave pulses to deliver to the vessel 130 from one or more shock wave emitters 190 of the IVL balloon, an IVL balloon pressure to use during a delivery of shock waves to the vessel 130 from the IVL balloon.

By way of another example, if the intravascular treatment device comprises a laser atherectomy catheter, the at least one parameter may include one or more of: a size of the laser atherectomy catheter, a location of the laser atherectomy catheter with respect to the plaque deposit 120, a fluence of optical irradiation emitted by the laser atherectomy catheter, a repetition rate of optical pulses emitted by the laser atherectomy catheter, a duty cycle of optical irradiation emitted by the laser atherectomy catheter, and an advancement speed of the laser atherectomy catheter.

By way of another example, if the intravascular treatment device comprises an orbital atherectomy device, the at least one parameter may include one or more of: an advancement speed of the orbital atherectomy device, a size of the orbital atherectomy device, a location of the orbital atherectomy device with respect to the plaque deposit 120, and a rotation speed of the orbital atherectomy device.

By way of another example, if the intravascular treatment device comprises a rotablation atherectomy device, the at least one parameter may include one or more of: a location of the rotablation atherectomy device with respect to the plaque deposit 120, an advancement speed or length of the rotablation atherectomy device, a retreatment length or speed of the rotablation atherectomy device, a burr size of the rotablation atherectomy device, and a rotation speed of the rotablation atherectomy device.

By way of another example, if the intravascular treatment device comprises a scoring or cutting balloon, the at least one recommended parameter may include one or more of: a location of the balloon with respect to the plaque deposit 120, a size of the balloon, an inflation pressure of the balloon, a blade length of the balloon, and an inflation time of the balloon.

The value(s) of the parameter(s) of the intravascular treatment device 180 may be determined from the plaque data 110 in various ways. In one example, a functional relationship between the value(s) of the parameter(s), and the plaque data 110, is used. The functional relationship may be provided by a graph, or a lookup table, for example. In another example, a biomechanical model is used to determine the value(s) of the parameter(s) of the intravascular treatment device 180 from the plaque data 110. In this example, the biomechanical model is constructed from the plaque data 110, and the biomechanical model is used to predict the effect of the parameter(s) of the intravascular treatment device 180 on the plaque deposit. For example, a finite element biomechanical model may be generated from the plaque data based on the measurement(s) of the plaque deposit and/or a composition of the plaque deposit. The biomechanical model may be generated based further on the lumen data and/or vessel wall data for the vessel 130. In another example, a neural network is used to determine the value(s) of the parameter(s) of the intravascular treatment device 180 from the plaque data 110. In this example, the neural network is trained to predict the value(s) of the parameter(s) from the plaque data 110. The neural network is trained to predict the value(s) of the parameter(s) using training data from historic intravascular plaque procedures. The training data includes plaque data 110 for the historic intravascular plaque procedures, and corresponding value(s) for the parameter(s) of the intravascular treatment device 180 that were used in procedures with successful outcomes.

An example is now described in which the intravascular treatment device 180 is an IVL balloon, and the values of various parameter(s) of the IVL balloon are determined from the plaque data 110 using a functional relationship, a biomechanical model, and a neural network.

A parameter of an IVL balloon that is typically determined during treatment is the number of shock wave pulses to deliver to the vessel 130 from the shock wave emitter(s) 190 of the IVL balloon. The number of shock wave pulses that are delivered by the IVL balloon affects the amount of damage that is inflicted upon a plaque deposit. The treatment of thick plaque deposits, or plaque deposits with a large angular extent, may require a relatively higher number of shock wave pulses than relatively thinner plaque deposits, or plaque deposits with a relatively smaller angular extent. The efficacy of the shock wave pulses that are delivered to a vessel has also been reported to be affected by the eccentricity of plaque deposits; shock wave pulses being more effective at fracturing plaque deposits that have a circular shape than plaque deposits that have an elliptical shape.

The number of shock wave pulses to deliver to the vessel 130 may be determined using a functional relationship such as a lookup table that relates the number of shock wave pulses to deliver to the vessel 130, to plaque data in the form of a thickness of the plaque deposit and/or an angular extent f of the plaque deposit 120 around a centerline 160 of the vessel 130 and/or an eccentricity of the plaque deposit. The lookup table may be generated from historic intravascular plaque procedures, and which include corresponding value(s) for the number of pulses that were used in procedures with successful outcomes.

Instead of using a functional relationship to determine the number of shock wave pulses to deliver to the vessel 130, a biomechanical model may be used. In this case, the biomechanical model of the plaque deposit 120 may be constructed from plaque data such as measurements of one or more of: a thickness of the plaque deposit, a depth of the plaque deposit from a centerline 160 of the vessel 130, an angular extent of the plaque deposit 120 around a centerline 160 of the vessel 130, and an eccentricity of the plaque deposit. Simulations may then be performed with the biomechanical model to determine the impact of a number of pulses from the shock wave emitters. The optimal number of shock wave pulses to deliver to the vessel 130 may then be determined based on a desired amount of damage to the plaque deposit 120.

Instead of using a biomechanical model to determine the number of shock wave pulses to deliver to the vessel 130, a neural network may be used. In this case, the neural network may be trained to predict the number of shock wave pulses to deliver to the vessel 130 from plaque data such as measurements of one or more of: a thickness of the plaque deposit, a depth of the plaque deposit from a centerline 160 of the vessel 130, an angular extent of the plaque deposit 120 around a centerline 160 of the vessel 130, and an eccentricity of the plaque deposit. The neural network may be trained to predict the number of pulses using training data from historic intravascular plaque procedures. The training data may include plaque data 110 for the historic intravascular plaque procedures, and corresponding value(s) for the number of pulses that were used in procedures with successful outcomes.

Another parameter of the IVL balloon that is typically determined during treatment is the IVL balloon pressure to use during the delivery of shock waves to the vessel 130 from the IVL balloon. The optimal balloon pressure is reported to be determined in-part by the thickness of the plaque deposit. Thus, the IVL balloon pressure may be determined using a functional relationship in the form of a lookup table that relates the balloon pressure, to plaque data in the form of a thickness of the plaque deposit.

Instead of using a functional relationship to determine the IVL balloon pressure, a biomechanical model may be used. In this case, the biomechanical model of the plaque deposit 120 may be constructed from plaque data such as measurements of one or more of: a thickness of the plaque deposit, a depth of the plaque deposit from a centerline 160 of the vessel 130, an angular extent of the plaque deposit 120 around a centerline 160 of the vessel 130, and an eccentricity of the plaque deposit. A biomechanical model of the IVL balloon within the biomechanical model of the plaque deposit 120, may then be used to simulate the effect of balloon pressure on its shape with respect to the plaque deposit 120. Simulations may then be performed with the two biomechanical models to determine the effect of balloon pressure on acoustic coupling between shock wave emitters within the IVL balloon and the plaque deposit in order to determine a value of the balloon pressure that provides optimal acoustic coupling.

Instead of using a biomechanical model to determine the IVL balloon pressure, a neural network may be used. In this case, the neural network may be trained to predict the IVL balloon pressure from plaque data such as measurements of one or more of: a thickness of the plaque deposit, a depth of the plaque deposit from a centerline 160 of the vessel 130, an angular extent of the plaque deposit 120 around a centerline 160 of the vessel 130, and an eccentricity of the plaque deposit. The neural network may be trained to predict the IVL balloon pressure using training data from historic intravascular plaque procedures. The training data may include plaque data 110 for the historic intravascular plaque procedures, and corresponding values for the IVL balloon pressure that was used in procedures with successful outcomes.

Another parameter of the IVL balloon that is typically determined during treatment is the size of the IVL balloon. The size of the IVL balloon should be such that it overlaps the plaque deposit along its length. Thus, the size of the IVL balloon may be determined using a functional relationship in the form of a lookup table that relates the size of the balloon, to plaque data in the form of an estimation of a length of the plaque deposit 120 along the vessel 130. The balloon should also have an inflated size that fits a plaque-free, or "healthy", lumen. Thus, the size of the IVL balloon may alternatively or additionally be determined using a functional relationship in the form of a lookup table that relates the size of the balloon, to lumen data in the form of a plaque-free measurement of an area of the lumen, or a minimum or maximum diameter of the lumen, or a measurement of a shape of the lumen.

Instead of using a functional relationship to determine the size of the IVL balloon, a biomechanical model may be used. In this case, the biomechanical model of the plaque deposit 120 may be constructed from plaque data such as measurements of one or more of: a thickness of the plaque deposit, a depth of the plaque deposit from a centerline 160 of the vessel 130, an angular extent of the plaque deposit 120 around a centerline 160 of the vessel 130, and an eccentricity of the plaque deposit. A biomechanical model of the IVL balloon within the biomechanical model of the plaque deposit 120, may then be used to simulate the effect of the size of the balloon on its shape with respect to the plaque deposit. The shape of the balloon may be simulated at various stages of its expansion. Simulations may then be performed with the two biomechanical models to determine the effect of the balloon size on the overlap between the balloon and the plaque deposit at the various stages of expansion of the balloon, and to thereby determine a balloon size that provides an optimal overlap.

Instead of using a biomechanical model to determine the IVL balloon size, a neural network may be used. In this case, the neural network may be trained to predict the IVL balloon size from plaque data such as measurements of one or more of: a thickness of the plaque deposit, a depth of the plaque deposit from a centerline 160 of the vessel 130, an angular extent of the plaque deposit 120 around a centerline 160 of the vessel 130, and an eccentricity of the plaque deposit. The neural network may be trained to predict the IVL balloon size using training data from historic intravascular plaque procedures. The training data may include plaque data 110 for the historic intravascular plaque procedures, and corresponding value(s) for the IVL balloon size that was used in procedures with successful outcomes.

Another parameter of the IVL balloon that is typically determined during treatment is the location of the IVL balloon with respect to the plaque deposit 120. In order to optimize the amount of damage that is delivered to the plaque deposit, the shock wave emitters 190 of the IVL balloon should be positioned such that they are optimally shielded by the plaque deposit. Thus, the location of the IVL balloon may be determined using a functional relationship in the form of a lookup table that relates the position of the balloon, to plaque data in the form of an estimation of a length of the plaque deposit 120 along the vessel 130. The location of the IVL balloon with respect to the plaque deposit 120 may be determined from plaque data such as the estimated length of the plaque deposit 120 along the vessel 130 by positioning the IVL balloon such that the emitters are within the estimated length of the plaque deposit 120 along the vessel 130.

Instead of using a functional relationship to determine the location of the IVL balloon with respect to the plaque deposit 120, a biomechanical model may be used. In this case, the biomechanical model may be constructed as described above for determining the size of the IVL balloon, and simulations performed with the two biomechanical models to determine the effect of the balloon location on the overlap between the balloon and the plaque deposit, in order to determine a balloon location that provides an optimal overlap. If the CT data 140 that is received in the operation S 110 includes spectral CT data, the biomechanical model of the plaque deposit may include a spatial distribution of calcified plaque that is determined from the spectral CT data. In this case, a balloon location is determined that provides an optimal overlap between the shock wave emitters in the balloon and the spatial distribution of the calcified plaque.

Instead of using a biomechanical model to determine the IVL balloon location, a neural network may be used. In this case, the neural network may be trained to predict the IVL balloon location from plaque data such as measurements of one or more of: a thickness of the plaque deposit, a depth of the plaque deposit from a centerline 160 of the vessel 130, an angular extent of the plaque deposit 120 around a centerline 160 of the vessel 130, and an eccentricity of the plaque deposit. For instance, the neural network may predict the optimal location with respect to the maximum thickness of the plaque deposit. The neural network may be trained to predict the IVL balloon location using training data from historic intravascular plaque procedures. The training data may include plaque data 110 for the historic intravascular plaque procedures, and corresponding value(s) for the IVL balloon location that was used in procedures with successful outcomes.

The values of parameters for other types of intravascular treatment devices may be determined in a similar manner.

In one example, the recommended value of the at least one parameter is determined based on a value of the corresponding at least one parameter from one or more similar historic intravascular plaque treatment procedures. In this example, the value(s) of the parameter(s) may be determined from the plaque data using a neural network, as described above. Alternatively, a database that includes data for one or more historic intravascular plaque treatment procedures may be used. If a database is used, the database may be queried in order to determine the recommended value(s) of the parameter(s). The one or more similar historic intravascular plaque treatment procedures may include at least one of:
a procedure performed using a similar type of intravascular treatment device;
a procedure performed in a similar type of vessel 130;
a procedure performed in a vessel having a similar geometry;
a procedure performed in a vessel having similar plaque data 110;
a procedure performed in a vessel having similar lumen data;
a procedure performed on a subject having a similar body mass index;
a procedure performed on a subject having the same gender;
a procedure performed on a subject having a similar age.

In this example, the database records for each historic procedure, the value(s) of the parameter(s) that were used for the procedure. The database may also record the type of intravascular treatment device, the type of vessel 130, the vessel geometry, the plaque data 110, the lumen data, the body mass index, the gender, the age, and so forth. The database may also record an associated value of an outcome metric for the procedure.

In this example, a similar historic intravascular plaque treatment procedure may be selected using a similarity metric which determines the similarity between the current procedure and the historic procedures based on the aforementioned factors. An example of a suitable similarity metric is the Mahalanobis distance. The value(s) of the parameter(s) for the current procedure may then be selected from the historic procedure having the highest value of the similarity metric.

In another example, the method described with reference to Fig. 9 also includes determining a value of an outcome metric for the intravascular plaque treatment procedure, based on the plaque data 110. In this example, the value of the outcome metric may represent factors such as a probability of success or failure of the procedure, or a probability of a medical complication occurring as a result of the procedure. The value of the outcome metric may be determined from the database described above by selecting the value of the outcome metric for the historic procedure having the highest value of the similarity metric. Alternatively, if a neural network is used to predicts the value(s) of the parameter(s), the neural network may be trained to predict the value of the outcome metric for the procedure. The neural network may be trained to predict the value of the outcome metric for the procedure by including in the training data, a corresponding value of an outcome metric for the procedure, and training the neural network to predict the value of the outcome metric. At inference, the neural network may predict the value(s) for the parameter(s) of the intravascular treatment device 180, as well as the value of the outcome metric.

In another example, a computer program product, is provided. The computer program product comprises instructions which when executed by one or more processors, cause the one or more processors to carry out a method of planning an intravascular plaque treatment procedure for a vessel 130. The method comprises:
executing S210 the operations S110, S120, S130, and S140;
determining S220 a recommended value of at least one parameter of an intravascular treatment device 180 for use in the intravascular plaque treatment procedure, based on the plaque data 110; and
outputting S230 the recommended value of the at least one parameter.

In another example, a system for planning an intravascular plaque treatment procedure for a vessel 130, is provided. The system includes one or more processors 210 configured to:
execute S210 the operations S110, S120, S130, and S140;
determine S220 a recommended value of at least one parameter of an intravascular treatment device 180 for use in the intravascular plaque treatment procedure, based on the plaque data 110; and
output S230 the recommended value of the at least one parameter.

In another example, a computer-implemented method of providing guidance during an intravascular plaque treatment procedure on a vessel 130, is provided. This method is described with reference to Fig. 10, which is a flowchart illustrating a first example of a computer-implemented method of providing guidance during an intravascular plaque treatment procedure, in accordance with some aspects of the present disclosure. The method described with reference to Fig. 10 may also be carried out by the one or more processors 310 of the system 300 illustrated in Fig. 11, which is a schematic diagram illustrating an example of a system 300 for providing guidance during an intravascular plaque treatment procedure, in accordance with some aspects of the present disclosure. With reference to Fig. 10, the computer-implemented method of providing guidance during an intravascular plaque treatment procedure on a vessel 130, includes:
executing S310 the operations S210, S220, and S230;
receiving S320 X-ray projection image data representing the vessel 130, the X-ray projection image data being generated during the intravascular plaque treatment procedure;
registering S330 the CT data 140 to the X-ray projection image data; and
outputting S340 a graphical representation of the X-ray projection image data and the CT data 140 as an overlay image.

In the operation S310, the operations S210, S220, and S230 that were described with reference to Fig. 9, are performed.

In the operation S320, X-ray projection image data representing the vessel 130, is received. The X-ray projection image data may be generated by an X-ray projection imaging system. The X-ray projection image data may be generated by the X-ray projection imaging system 320 illustrated in Fig. 11, for example. The X-ray projection image data may represent a static image, of the vessel or a temporal sequence of images of the vessel. In the latter case, the temporal sequence of images may represent the vessel substantially in real-time. The operations of receiving S320, registering S330, and outputting S340 may be performed substantially in real-time in order to provide live guidance during the intravascular treatment procedure on the vessel 130. The X-ray projection image data may be received by the one or more processors 310 illustrated in Fig. 11, for example.

An X-ray projection imaging system typically includes a support arm such as a so-called "C-arm" that supports an X-ray source and an X-ray detector. X-ray projection imaging systems may alternatively include a support arm with a different shape to this example, such as an O-arm, for example. In contrast to a CT imaging system, an X-ray projection imaging system generates attenuation data for an object with the X-ray source and X-ray detector in a static position with respect to the object. An example of an X-ray projection imaging system that may be used to generate projection attenuation data 110 that is received in the operation S110, is the Azurion 7 X-ray projection imaging system that is marketed by Philips Healthcare, Best, The Netherlands.

In the operation S330, the CT data 140 is registered to the X-ray projection image data. The operation S330 may be performed using known image registration techniques. Examples of suitable image registration techniques include intensity-based registration techniques, feature-based registration techniques, rigid and non-rigid registration techniques, and so forth.

In the operation S340, a graphical representation of the X-ray projection image data and the CT data 140, is outputted as an overlay image. The operation S340 may be performed using known image overlay techniques. For instance, one image from the X-ray projection image data and the CT data 140 may be overlaid on the other image, wherein the overlaid image has a predetermined level of transparency. The graphical representation may be outputted in various ways, such as to display. The graphical representation may be outputted to the display 330 illustrated in Fig. 11, for example. The outputted overlay image provides guidance to a physician during an intravascular plaque treatment procedure on the vessel 130.

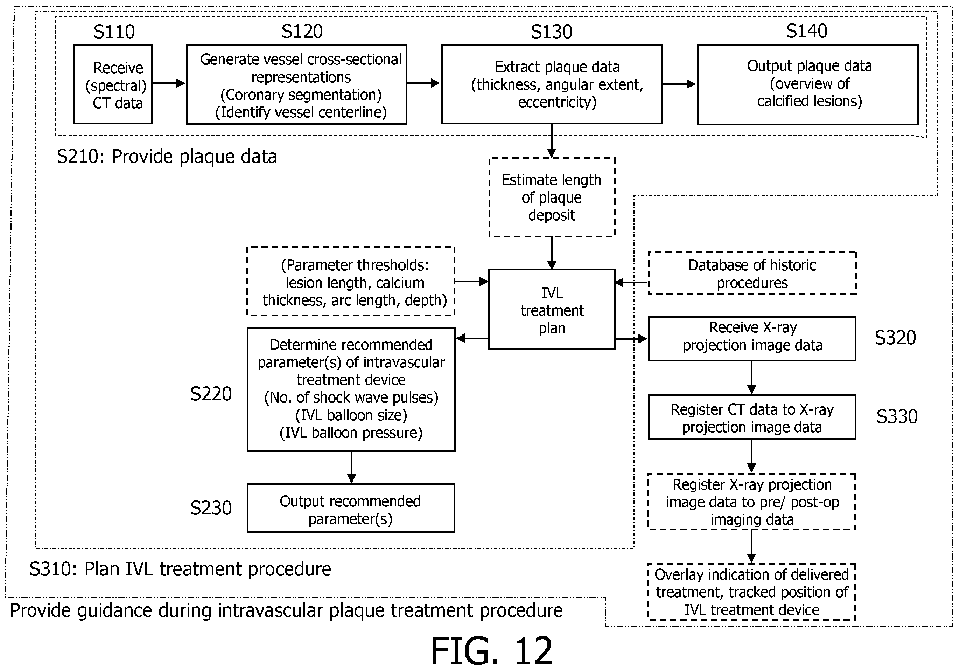

Fig. 12 is a flowchart illustrating a second example of a computer-implemented method of providing guidance during an intravascular plaque treatment procedure, in accordance with some aspects of the present disclosure. The flowchart illustrated in Fig. 12 indicates the dependency of the method of providing guidance during an intravascular plaque treatment procedure, i.e. the method described with reference to Fig. 10, on the method of planning an intravascular plaque treatment procedure for a vessel 130, i.e. the operation S310, and the method of providing plaque data 110 for a plaque deposit 120 in a vessel 130, i.e. the operation S210.

In one example, an indication of the delivery of the treatment to the vessel 130, is included in the overlay image. In this example, the method of providing guidance during an intravascular plaque treatment procedure described with reference to Fig. 10, includes:
receiving input data representing a delivery of a treatment to the vessel 130 by the intravascular treatment device; and
updating the overlay image based on the received input data; and
wherein the updating comprises providing in the overlay image an indication of the delivery of the treatment to the vessel 130.

The indication of the delivery of the treatment to the vessel 130 may indicate the position at which the treatment was delivered and/or a difference between a planned delivery of the treatment and an actual delivery of the treatment.

This example may be performed by tracking the position of the intravascular treatment device with respect to the X-ray projection image data that is generated during the intravascular plaque treatment procedure, and indicating the tracked position of the intravascular treatment device in the overlay image at the times at which the treatment is delivered to the vessel. By way of an example, the indication of the delivery of the treatment to the vessel 130 may indicate that a specified number of pulses have been delivered to the vessel 130 by the one or more shock wave emitters 190 of the IVL balloon 180 illustrated in Fig. 8. The indication of the delivery of the treatment to the vessel 130 may be provided in the overlay image as an icon at the relevant position, for example. The tracked position of the treatment device may be determined in the X-ray projection image data, or using a separate tracking system. In the former case, the tracked position of the intravascular treatment device may be determined in the X-ray projection image data using a feature detector that is trained to detect the shape of the intravascular treatment device. Alternatively, a feature detector may be trained to detect the shape of a fiducial marker that is attached to the treatment device. In the latter case, various interventional device tracking techniques may be used to track the position of the intravascular treatment device. The position may be determined in the X-ray projection image by registering the coordinate system of the tracking system to the coordinate system of the X-ray projection imaging system 320 that generates the X-ray projection image data. Various tracking systems may be used to track the position of the intravascular treatment device, including electromagnetic tracking systems and optical fiber-based tracking systems. An example of an electromagnetic tracking system is disclosed in the document US 2020/397510 A1. An example of an optical fiber-based tracking technique that uses a strain sensor to determine the position of an interventional device is disclosed in the document US 2012/323115 A1.

In this example, the received input data may represent a position of the delivery of the treatment to the vessel 130. The operation of updating the overlay image may include providing in the overlay image an indication of the position of the delivery of the treatment to the vessel 130. Alternatively, or additionally, the operation of updating the overlay image may include providing in the overlay image an indication of a total number of treatments delivered to the vessel 130. Thus, the overlay image provides a record of the treatment that has been delivered to the vessel.

In another example, a computer program product, is provided. The computer program product comprises instructions which when executed by one or more processors, cause the one or more processors to carry out a method of providing guidance during an intravascular plaque treatment procedure on a vessel 130. The method comprises:
executing S310 the operations S210, S220, and S230;
receiving S320 X-ray projection image data representing the vessel 130, the X-ray projection image data being generated during the intravascular plaque treatment procedure;
registering S330 the CT data 140 to the X-ray projection image data; and
outputting S340 a graphical representation of the X-ray projection image data and the CT data 140 as an overlay image.

In another example, a system for providing guidance during an intravascular plaque treatment procedure on a vessel 130, is provided. The system includes one or more processors 210 configured to:
execute S310 the operations S210, S220, and S230;
receive S320 X-ray projection image data representing the vessel 130, the X-ray projection image data being generated during the intravascular plaque treatment procedure;
register S330 the CT data 140 to the X-ray projection image data; and
output S340 a graphical representation of the X-ray projection image data and the CT data 140 as an overlay image.

The above examples are to be understood as illustrative of the present disclosure, and not restrictive. Further examples are also contemplated. For instance, the examples described in relation to computer-implemented methods, may also be provided by the corresponding computer program product, or by the corresponding computer-readable storage medium, or by the corresponding system. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of providing plaque data (110) for a plaque deposit (120) in a vessel (130), the method comprising:
receiving (S110) computed tomography, CT, data (140) representing the vessel (130);
generating (S120), from the CT data (140), a cross-sectional representation (150) of the vessel (130) at each of a plurality of positions (A - A', B - B') along the vessel;
extracting (S130), from the cross-sectional representations, plaque data (110) comprising at least one measurement of the plaque deposit (120) at the plurality of positions along the vessel; and
outputting (S140) a graphical representation of the plaque data (110).

2. The computer-implemented method according to claim 1, wherein the generating (S120) a cross-sectional representation (150) of the vessel (130) at each of a plurality of positions (A - A', B - B') along the vessel, comprises:
identifying, from the CT data (140), a centerline (160) of the vessel (130), and wherein the cross-sectional representations of the vessel are generated at the plurality of positions along the centerline (160) of the vessel.

3. The computer-implemented method according to claim 2, wherein the identifying a centerline (160) of the vessel (130), comprises determining a centerline of the lumen (170) of the vessel, and defining the centerline (160) of the vessel (130) as the centerline of the lumen (170).

4. The computer-implemented method according to claim 1, wherein the at least one measurement of the plaque deposit (120) comprises at least one of: a thickness of the plaque deposit, a depth of the plaque deposit from a centerline (160) of the vessel (130), an angular extent (f) of the plaque deposit (120) around a centerline (160) of the vessel (130), and an eccentricity of the plaque deposit.

5. The computer-implemented method according to claim 1, wherein the plaque data (110) further comprises an estimation of a length of the plaque deposit (120) along the vessel (130); and
wherein the estimation of the length of the plaque deposit (120) along the vessel (130) is calculated based on a separation between the positions along the vessel corresponding to the proximal and distal ends of a contiguous set of cross-sectional representations in which at least one measurement of the plaque deposit (120) exceeds a predetermined value.

6. The computer-implemented method according to any previous claim, wherein the method further comprises:
extracting, from the cross-sectional representations, lumen data and/or vessel wall data for the vessel (130), the lumen data comprising at least one measurement of a lumen (170) of the vessel (130), and the vessel wall data comprising at least one measurement of the wall of the vessel (130), at the plurality of positions (A - A', B - B') along the vessel.

7. The computer-implemented method according to claim 6, wherein the method further comprises:
estimating, for cross-sectional representations wherein at least one measurement of the plaque deposit (120) exceeds a threshold value, a plaque-free measurement of the lumen (170) at the corresponding position along the vessel (130); and
wherein the estimated plaque-free measurement of the lumen (170) is determined based on the corresponding measurement of the lumen from a neighboring cross-sectional representation wherein the at least one measurement of the plaque deposit (120) is below the threshold value.

8. The computer-implemented method according to any previous claim, wherein the CT data (140) comprises spectral CT data defining X-ray attenuation of the vessel (130) in a plurality of different energy intervals (DE_{1..m}); and
wherein the extracting (S130) plaque data (110), and/or the determining a centerline of the lumen (170) of the vessel (130), and/or the extracting lumen data for the vessel (130), comprises applying a material decomposition algorithm to the spectral CT data.

9. The computer-implemented method according to claim 8, wherein the extracting (S130) plaque data (110) comprises applying a material decomposition algorithm to the spectral CT data to identify a type of the plaque deposit (120) at the plurality of positions (A - A', B - B') along the vessel (130).

10. A computer-implemented method of planning an intravascular plaque treatment procedure for a vessel (130), the method comprising:
executing (S210) the method of providing plaque data according to any one of claims 1 - 9;
determining (S220) a recommended value of at least one parameter of an intravascular treatment device (180) for use in the intravascular plaque treatment procedure, based on the plaque data (110); and
outputting (S230) the recommended value of the at least one parameter.

11. The computer-implemented method according to claim 10, wherein:
the intravascular treatment device (180) comprises an intravascular lithotripsy, IVL, balloon, and wherein the at least one parameter comprises one or more of: a size of the IVL balloon, a location of the IVL balloon with respect to the plaque deposit (120), a number of shock wave pulses to deliver to the vessel (130) from one or more shock wave emitters (190) of the IVL balloon, an IVL balloon pressure to use during a delivery of shock waves to the vessel (130) from the IVL balloon; or
the intravascular treatment device comprises a laser atherectomy catheter, and wherein the at least one parameter comprises one or more of: a size of the laser atherectomy catheter, a location of the laser atherectomy catheter with respect to the plaque deposit (120), a fluence of optical irradiation emitted by the laser atherectomy catheter, a repetition rate of optical pulses emitted by the laser atherectomy catheter, a duty cycle of optical irradiation emitted by the laser atherectomy catheter, and an advancement speed of the laser atherectomy catheter; or
the intravascular treatment device comprises an orbital atherectomy device, and wherein the at least one parameter comprises one or more of: an advancement speed of the orbital atherectomy device, a size of the orbital atherectomy device, a location of the orbital atherectomy device with respect to the plaque deposit (120), and a rotation speed of the orbital atherectomy device; or
the intravascular treatment device comprises a rotablation atherectomy device, and wherein the at least one parameter comprises one or more of: a location of the rotablation atherectomy device with respect to the plaque deposit (120), an advancement speed or length of the rotablation atherectomy device, a retreatment length or speed of the rotablation atherectomy device, a burr size of the rotablation atherectomy device, and a rotation speed of the rotablation atherectomy device; or
the intravascular treatment device comprises a scoring or cutting balloon, and wherein the at least one parameter comprises one or more of: a location of the balloon with respect to the plaque deposit (120), a size of the balloon, an inflation pressure of the balloon, a blade length of the balloon, and an inflation time of the balloon.

12. The computer-implemented method according to claim 11, wherein the recommended value of the at least one parameter is determined based further on a value of the corresponding at least one parameter from one or more similar historic intravascular plaque treatment procedures.

13. The computer-implemented method according to claim 12, wherein the one or more similar historic intravascular plaque treatment procedures comprise at least one of:
a procedure performed using a similar type of intravascular treatment device;
a procedure performed in a similar type of vessel (130);
a procedure performed in a vessel having a similar geometry;
a procedure performed in a vessel having similar plaque data (110);
a procedure performed in a vessel having similar lumen data;
a procedure performed on a subject having a similar body mass index;
a procedure performed on a subject having the same gender;
a procedure performed on a subject having a similar age.

14. A computer-implemented method of providing guidance during an intravascular plaque treatment procedure on a vessel (130), the method comprising:
executing (S310) the method of planning an intravascular plaque treatment procedure according to any one of claims 10 - 12, and wherein the method further comprises:
receiving (S320) X-ray projection image data representing the vessel (130), the X-ray projection image data being generated during the intravascular plaque treatment procedure;
registering (S330) the CT data (140) to the X-ray projection image data;
and
outputting (S340) a graphical representation of the X-ray projection image data and the CT data (140) as an overlay image.

15. The computer-implemented method according to claim 14, wherein the method further comprises:
receiving input data representing a delivery of a treatment to the vessel (130) by the intravascular treatment device; and
updating the overlay image based on the received input data; and
wherein the updating comprises including in the overlay image an indication of the delivery of the treatment to the vessel (130).
